# EUROPEAN PATENT APPLICATION

(11) **EP 1 300 162 A1**
(43) Date of publication of application: **09.04.2003**
(21) Application number: 99944649.5
(22) Date of filing: 10.09.1999
(51) Int. Cl.: A61L 9/03

(54) **ELECTRICAL DIFFUSER FOR AIR FRESHENERS, INSECTICIDES AND THE LIKE**

(71) Applicant: DBK Espana, S.A., 08290 Cerdanyola del Valles (ES)
(72) Inventor: BASAGANAS MILLAN, Jordi Argenters, 2-4-8 Edif.3C/P, 08290 Barcelona (ES)
(74) Representative: Carpintero Lopez, Francisco
(86) International application number: ES9900287
(87) International publication number: WO01019416

(57) **Abstract**

An electrical diffuser is disclosed which evaporates air fresheners, insecticides or the like, which operates automatically depending on the ambient light intensity, so that when it is used for air fresheners the diffuser operates during the daytime and when used for insecticides it operates at night and disconnects when daytime arrives. Said automatic operation is achieved by means of an electronic circuit provided with a light sensor which operates an electronic switch for the typical electrical resistor which causes evaporation of the product.

## Description

### OBJECT OF THE INVENTION

The present invention relates to an electrical diffuser, of those which cause the evaporation or sublimation of an air freshener, insecticide or the like, diffuser which is designed and structured in order to obtain an automatic operation limited to the moments in which its operation is more necessary, according to the type of product diffused.

### BACKGROUND OF THE INVENTION

Diffusers are known which, with an air freshener, insecticide, etc. as the raw matter, are provided with a heating device, specifically an electrical resistance, which supplies the required heat to the product, whether in solid or liquid state, in order to establish the suitable rate of evaporation or sublimation.

More specifically, these devices are provided with a body in which is generally established the plug for the electrical power supply to the heating element, inside which is a housing either for a pill of solid product or for the container of the liquid product, with the corresponding evaporation wick.

Their design is generally such that the electrical plug acts as a wall support for the diffuser within the socket chosen for it in a room, so that its mechanical attachment brings about an electrical connection in parallel, with in the best of cases a switch provided to disconnect the power supply from the heating element without having to unplug the device from its place of operation.

This implies that either the device is permanently connected, twenty-four hours a day, as often occurs, or one must remember to connect and disconnect it when desired or when it is considered necessary, as if the diffuser is used as an air freshener there is no sense in having it operate at night and if it is used as an insecticide it is not required during the day, as mosquitoes generally act at night-time.

### DESCRIPTION OF THE INVENTION

The electrical diffuser disclosed by the invention has been conceived and designed in order to solve the above problems to a full satisfaction, allowing an automatic operation with an automatic electrical connection and disconnection depending on the luminous intensity of the surroundings.

For this purpose, and more specifically, based on the structure of any conventional electrical diffuser, that is, employing an electrical heating resistance provided with means for its connection to the electricity network of any home or premises, the diffuser of the invention centers its characteristics in that it incorporates a light sensor with the associated electronic circuitry, which acts as a switch for the heating element so that, depending on the signal received by said light sensor, the diffuser will operate or not.

More specifically, when the diffuser is meant for air fresheners said electronic switch shall cause the connection of the heating element in the presence of light, so that the diffuser operates during the daytime and is off during the night, while when it is meant to diffuse insecticides its operation is the inverse, that is, the absence of light resulting in connection and its presence the disconnection, so that the diffuser operates during the daytime and is off during the night.

### DESCRIPTION OF THE DRAWINGS

As a complement of the description and in order to aid a better understanding of the characteristics of the invention, according to an example of a preferred embodiment, the memory is accompanied by a set of drawings where, for purposes of illustration and in a non-limiting sense the following is represented:

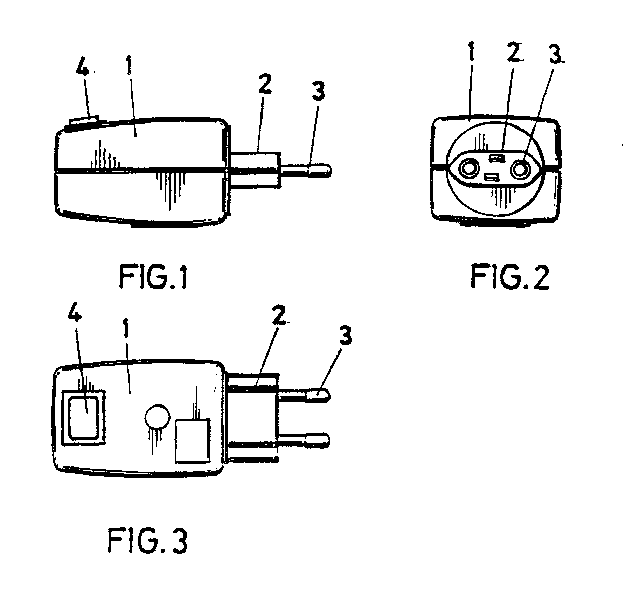

Figure 1.- Shows, in a side elevation view, an electrical diffuser for air fresheners, insecticides and the like according to the object of the invention.
Figure 2.- Shows an axial view of the same diffuser.
Figure 3.- Shows, finally, a plan view of the same diffuser.

### PREFERRED EMBODIMENT OF THE INVENTION

In view of these figures the electrical diffuser disclosed is shown to comprise, as any conventional diffuser of this type, a body (1) structured according to any design lines and from which emerges, as a single part, a plug (2) with pins (3), which in addition to being the means for electrical connection of the diffuser circuit also serve as the means of attachment to the wall electrical socket.

Based on this basic and conventional structure, the electrical diffuser disclosed by the invention centers its characteristics in that it incorporates a light sensor (4) with the associated electronic circuitry, which acts as a switch for the classical electrical resistor or heating which supplies the product pill or the product container the heat required to diffuse said product.

Thus, according to the above, connection and disconnection of the heating element occurs automatically depending on the luminosity of the surroundings sensed by light sensor (4), without prejudice of the electrical diffuser being unplugged in a conventional manner by extracting pins (3) from the socket.

Based on this basic structure and depending on the specific application of the diffuser, the electronic circuit which comprises the switch may differ slightly, so that as mentioned above, when the diffuser is meant fcr air fresheners said electronic switch shall close the power supply circuit for the heating element in the presence of light, that is, when light sensor (4) is excited, so that the diffuser operates during the daytime, while when it is meant to diffuse insecticides its operation is the inverse, that is, the presence of light resulting in an open circuit and light sensor (4) closing the circuit when it detects the presence of light, so that the diffuser operation is limited to the night-time, which is when the insecticide product is required.

Lastly, for said electronic circuitry comprising the switch excited by the light sensor (4) there are a number of conventional solutions which may be used for other purposes, any of which can in principle be valid as long as its baseplate size is suitable for the capacity of case (1) and/or said case is adapted to said printed circuit board.

## Claims

1. Electrical diffuser for air fresheners, insecticides and the like, of the type which include a heating element embodied as an electrical resistor, suitably arranged in order to supply heat to either a solid product pill, or a container of a liquid product, so that the heat generated by said heating element causes evaporation or sublimation of the product concerned, **characterised in that** it incorporates an electronic switch automatically operated by a light sensor, so that the operation of the diffuser depends on the ambient light intensity.

2. Electrical diffuser for air fresheners, insecticides and the like, as claimed in claim 1, **characterised in that** the electronic switch closes the power supply circuit of the heating element when a certain ambient light intensity is exceeded.

3. ^{a}.- Electrical diffuser for air fresheners, insecticides and the like, as claimed in claim 1, **characterised in that** the electronic switch closes the power supply circuit of the heating element when under a certain ambient light intensity.
